# EUROPEAN PATENT APPLICATION

(11) **EP 3 431 027 A1**
(43) Date of publication of application: **23.01.2019**
(21) Application number: 17766914.0
(22) Date of filing: 06.03.2017
(51) Int. Cl.: A61B 17/72, A61B 17/86

(54) **INTRAMEDULLARY NAIL HAVING ANTI-TILT FUNCTION**

(30) Priority: 18.03.2016 KR 20160032870
(71) Applicant: Lee, Dong Hoon, Seoul 05109 (KR)
(72) Inventor: Lee, Dong Hoon, Seoul 05109 (KR)
(74) Representative: Reiser & Partner Patentanwälte mbB
(86) International application number: PCT/KR2017/002408
(87) International publication number: WO 2017/160013

(57) **Abstract**

An intramedullary nail having an anti-tilt function and an overall length of L comprises one or more bores formed in each of a proximal region located at an upper part of the intramedullary nail and a distal region located at a lower part thereof, wherein: one or more first bores are formed in a first region located at a lower side of the proximal region, and the range of the first region is 0.12 L to 0.45 L from a proximal end portion; and one or more second bores are formed in a second region located at an upper side of the distal region, and the range of the second region is 0.46 L to 0.85 L from the proximal end portion.

## Description

### TECHNICAL FIELD

The present invention relates to an intramedullary nail, and more particularly, to an intramedullary nail having bores in a specific region in order for a segmented bone (including a fractured bone due to an external force and a cut bone for distraction osteogenesis) to maintain an upright position without inclination or deflection.

### BACKGROUND

First, as for a structure of a bone, there is a cavity called a medullary cavity at the center of the bone, and the medullary cavity is filled with bone marrow which is a hematopoietic tissue. A cavernous body in a spongy form is formed outside of the medullary cavity, a compact substance is formed outside of the cavernous body, and an outside of the compact substance, i.e., the outermost surface of the bone, is covered by periosteum which is thin and tough.

The intramedullary nail of the present invention can be inserted into the medullary cavity for support and smooth bond of a bone when the bone is segmented. In particular, such an intramedullary nail is often used for distraction osteogenesis of the large tubular bones of the upper leg (femur) or shin bone (tibia).

In this connection, Patent Document 1 discloses an intramedullary nail having a central portion comprised of several curved portions. The intramedullary nail disclosed in the Patent Document 1 is characterized in that it has two curvatures having different radii to each other so that it could be optimally received within a medullary cavity.

However, since a medullary cavity portion into which the intramedullary nail is inserted is a space comprised of a soft tissue, and muscles around a segmented bone push or pull the bone, the bone is not being fixed and tends to be tilted in a specific direction when s conventional nail is inserted into the medullary cavity. Furthermore, when the bone is bonded in a tilted state, its shape may deviate from an original shape or targeted shape of the bone, resulting in various complications, a decreased capability of bearing a body weight, or decreased motor ability.

### DETAILED DESCRIPTION OF THE INVENTION

### TECHNICAL OBJECTS

The present invention has been made to solve the above-mentioned problems of the conventional art, and it is an object of the present invention to provide an intramedullary nail which can prevent a bone from tilting in a specific direction when it is inserted into a medullary cavity of a segmented bone.

It is another object of the present invention to provide an intramedullary nail which can guide a bone in an intended direction until a bone formation is completed and a segmented bone is finally bonded.

In addition, it is still another object of the present invention to minimize complications, and to allow a bonded bone to support a body weight properly while minimizing the decrease of motor ability, by using the intramedullary nail of the present invention for bonding a segmented bone.

The object of the present invention is not limited to the above-mentioned technical problems and other technical problems which are not mentioned are clearly understood from the following description to those skilled in the art to which the present invention belongs.

### SOLUTION OF PROBLEMS

In order to achieve the above objects, according to an aspect of the present invention, there is provided an intramedullary nail having an overall length of L, the nail comprising one or more bores formed in each of a proximal region located in an upper portion of the intramedullary nail and a distal region located in a lower portion of the intramedullary nail, wherein one or more first bores are formed in a first region located in a lower side of the proximal region and a range of the first region is 0.12L to 0.45L from a proximal end of the intramedullary nail, and wherein one or more second bores are formed in a second region located in an upper side of the distal region and a range of the second region is 0.46L to 0.85L from the proximal end.

According to another aspect of the present invention, there is provided an intramedullary nail, wherein the range of the first region is 0.17L to 0.4L from the proximal end, and the range of the second region is 0.51L to 0.8L from the proximal end.

According to yet another aspect of the present invention, there is provided an intramedullary nail, wherein the intramedullary nail has a through hole formed to extend from the proximal end toward a distal end of the intramedullary nail, and the first bore and the second bore are configured to communicate with the through hole.

According to yet another aspect of the present invention, there is provided an intramedullary nail, wherein the bores including the first bore and the second bore are formed radially with respect to a central axis.

Further, according to yet another aspect of the present invention, there is provided an intramedullary nail, wherein at least one of the bores including the first bore and the second bore is formed to have a predetermined angle with respect to a face of the proximal end.

According to yet another aspect of the present invention, there is provided an intramedullary nail, wherein at least two of the bores including the first bore and the second bore are formed so as not to be parallel to each other.

According to yet another aspect of the present invention, there is provided an intramedullary nail, wherein at least one of the bores including the first bore and the second bore is a Y-shaped bore forming three openings on an outer circumferential surface of the intramedullary nail.

According to yet another aspect of the present invention, there is provided an intramedullary nail, wherein at least one of the bores including the first bore and the second bore is a bore having a longitudinally-elongated shape.

### EFFECTS OF THE INVENTION

According to an aspect of the present invention, it is possible to prevent a segmented bone from tilting (i.e., inclining in a specific direction) due to the action of nearby muscles, with an intramedullary nail inserted into the medullary cavity of the segmented bone.

In addition, the intramedullary nail according to the present invention can guide a bone in an intended direction until a bone formation is completed and the segmented bone is finally bonded.

In addition, it is possible to minimize complications, and for a bonded bone to support a body weight properly while minimizing the decrease of motor ability, by using the intramedullary nail of the present invention for bonding a segmented bone.

It should be understood that the effects of the present invention are not limited to the above effects and include all effects that can be deduced from the detailed description of the present invention or the constitutions of the invention described in the appended claims.

### BRIEF DESCRIPTION OF THE DRAWINGS

Fig. 1 is a schematic diagram illustrating a tilting of a bone that occurs when a conventional intramedullary nail is inserted into the bone.
Fig. 2 is a front view illustrating an intramedullary nail according to an embodiment of the present invention, which is inserted into a segmented bone.
Fig. 3 is a side view of an intramedullary nail according to an embodiment of the present invention.
Fig. 4 is a schematic view illustrating a bone which is completely bonded, with an intramedullary nail according to an embodiment of the present invention inserted into the bone.

### DETAILED DESCRIPTION OF THE INVENTION

Hereinafter, the present invention will be described with reference to the accompanying drawings. The present invention may, however, be embodied in many different forms and should not be construed as limited to the embodiments set forth herein. For the purpose of clarity, the parts not related to the description are omitted, and analogous reference numerals are used among the drawings to indicate corresponding or analogous elements.

In the specification, when a part is "connected" to another part, it is not only "directly connected", but also "indirectly connected" via another element therebetween. Also, when an element is referred to as "comprising" any constituents, it means that it can include other constituents unless specifically stated otherwise.

Hereinafter, embodiments of the present invention will be described in detail with reference to the accompanying drawings.

In the medical field, break of a bone by an external force applied to the bone due to an accident or the like is referred to as a 'bone fracture', and breaking a specific part of a bone intentionally for distraction osteogenesis or the like is referred to as a 'bone cutting'. In this specification, 'segmentation' will be used as a term including both the concepts of 'fracture' and 'cutting'.

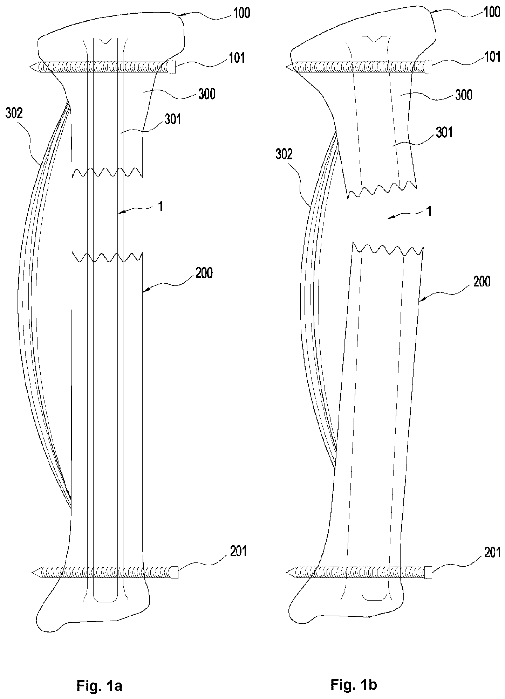

Fig. 1 is a schematic diagram illustrating tilting of a bone that occurs when a conventional intramedullary nail 1 is inserted into the bone. The bone can be femur or tibia in particular. In Fig. 1, the tibia will be described as an example. Fig. 1(a) illustrates a conventional intramedullary nail 1 which is inserted into a medullary cavity 301 of the tibia. The medullary cavity 301 is a soft tissue, and outside of the soft tissue is surrounded by a hard part 300. A segmented tibia can be distinguished by upper tibia 100 and lower tibia 200, and the upper tibia 100 and the lower tibia 200 are connected by muscles 302 around the tibia. Fig. 1(b) illustrates the upper tibia 100 inclined in a counterclockwise direction and the lower tibia 200 inclined in a clockwise direction. Such an inclining phenomenon of the upper tibia 100 and the lower tibia 200 is referred to as tilting, and bolts 101 and 201 could be bent or a part of the bone around the bolts 101 and 201 could be broken in the process of tilting.

Tilting of the upper tibia 100 and the lower tibia 200 is caused by the following reasons: Firstly, although the intramedullary nail is inserted into the medullary cavity 301, the upper and lower tibiae 100 and 200 may move until the intramedullary nail contacts the hard part 300 since the medullary cavity 301 is a soft tissue. Secondly, the muscles 302 around the tibia tends to move the position of the upper and lower tibiae 100 and 200. Thirdly, since the upper tibia 100 is coupled to an intramedullary nail 2 only at its upper side, and the lower tibia 200 is coupled to the intramedullary nail 2 only at its lower side, a lower side of the upper tibia 100 and an upper side of the lower tibia 200 can freely move and thus cannot resist the force of the muscles 302. When tilting of the upper tibia 100 and the lower tibia 200 occurs, the bones are bonded in an unintended direction so that complications may occur after the bonding, the bonded bone may not properly support a body weight, and there is a high possibility of degradation of motor ability.

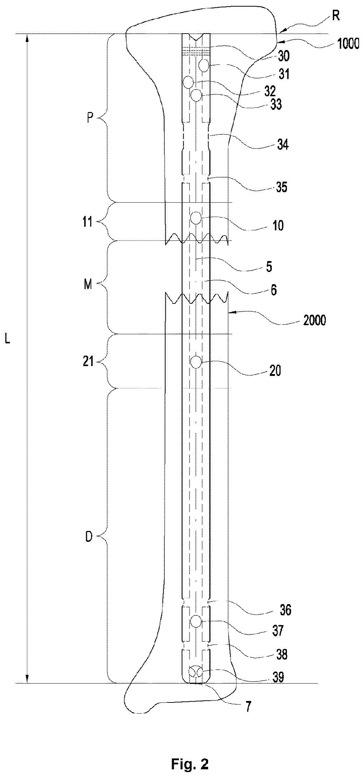

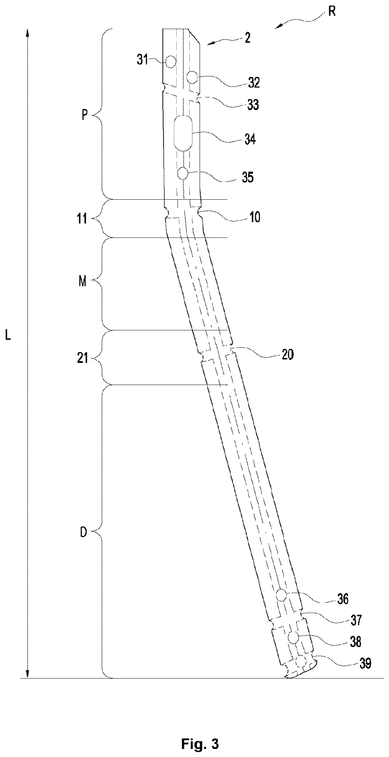

Fig. 2 is a front view of an intramedullary nail according to an embodiment of the present invention, in which the intramedullary nail is inserted into a segmented bone, and Fig. 3 is a side view of an intramedullary nail according to an embodiment of the present invention. As illustrated in Figs. 2 and 3, the intramedullary nail 2 of the present invention which is inserted into the medullary cavity can support and guide the bone until the segmented bone is boned. The intramedullary nail 2 has an overall length L and has a proximal region P, a central region M, a distal region D, a first region 11 and a second region 21.

The proximal region P is located in the upper part of the intramedullary nail 2, and the distal region D is located in the lower part of the intramedullary nail 2. The first region 11 is located on a lower side of the proximal region P, and the second region 21 is located on an upper side of the distal region D.

More specifically, the proximal region P is a region between a proximal end 4 of the intramedullary nail 2 and the first region 11, the central region M is a region between the first region 11 and the second region 21, and the distal region D is a region between the second region 21 and a distal end 7 of the intramedullary nail 2.

A bore 30 in the form of a female screw extending in a longitudinal direction may be formed in the intramedullary nail 2 between 0.017L and 0.06L from the proximal end 4 of the intramedullary nail 2. The bore 30 may receive an additional brace (not shown) capable of supporting the intramedullary nail 2.

At least one bore may be formed in the proximal region P of the intramedullary nail 2. Fig. 2 shows a total of five bores 31, 32, 33, 34 and 35 formed in the proximal region P, for example. The intramedullary nail 2 and an upper tibia 1000 can be coupled by bolts through the bores 31, 32, 33, 34 and 35 and the deviation of the upper part of the upper tibia 1000 from the intramedullary nail 2 can be prevented.

Also, one or more bores may be formed in the distal region D of the intramedullary nail 2. Fig. 2 illustrates a total of four bores 36, 37, 38 and 39 formed in the distal region D, for example. The intramedullary nail 2 and a lower tibia 2000 can be coupled by bolts through the bores 36, 37, 38 and 39 and the deviation of the lower part of the lower tibia 1000 from the intramedullary nail 2 can be prevented.

When the intramedullary nail 2 is used for distraction osteogenesis, the total length L of the intramedullary nail 2 may be 250 mm to 300 mm. In the distraction osteogenesis, a segmented area may range from 100 mm to 200 mm from the proximal end 4, which corresponds to an area between 0.33L and 0.8 L when converted to the overall length L basis. More preferably, the segmented area can range from 100 mm to 180 mm from the proximal end 4, which corresponds to an area between 0.33L and 0.72L.

The first region 11 may range from 0.12L to 0.45L from the proximal end. At least one first bore 10 may be formed in the first region 11 of the intramedullary nail 2. The first bore 10 can engage with a bolt (not shown). The first region 11 is always located in an upper side of the segmented area in the direction of the proximal end 4, and thus, the first bore 10 formed in the first region 11 is also always located in the upper side of the segmented area in the direction of the proximal end 4. Further, even when the number of the first bore 10 is plural, all of the plurality of first bores 10 are always located in the upper side of the segmented area in the direction of the proximal end 4.

Thus, as illustrated in Fig. 2, all of the bores formed in the proximal region P and the first bore 10 formed in the first region 11 can be positioned to correspond to the upper tibia 1000. In addition, with this bore arrangement, the bores (e.g., 31, 32, 33, 34 and 35 in Fig. 2) formed in the proximal region P can couple the upper portion of the upper tibia 1000 with intramedullary nail 2, and the first bore 10 formed in the first region 11 can couple the lower portion of the upper tibia 1000 with the intramedullary nail 2. Preferably, the above coupling may be a bolt coupling.

When both the upper and lower portions of the upper tibia 1000 are coupled with the intramedullary nail 2, the upper tibia 1000 is supported by at least two points in the longitudinal direction. With this supporting method, tilting of the upper tibia 1000 caused by the muscles around the upper tibia 1000 can be prevented since the upper tibia 1000 cannot be rotated.

Furthermore, since the number of the bore formed in the proximal region P and the number of the first bore 10 formed in the first region 11 may be plural, the upper and lower portions of the upper tibia 1000 can be fixed to the intramedullary nail 2 more firmly, thereby even a minute tilting of the upper tibia 1000 can be prevented.

The range of the second region 21 may be 0.46L to 0.85L from the proximal end. At least one second bore 20 may be formed in the second region 21 of the intramedullary nail 2. The second bore 20 can engage with a bolt (not shown). The second region 21 is always located in a lower side of the segmented area in the direction of the distal end 7, and thus, the second bore 20 formed in the second region 21 is also always located in the lower side of the segmented area in the direction of the distal end 7. Furthermore, even when the number of the second bore 20 is plural, all of the plurality of second bores 20 are always positioned in the lower side of the segmented region in the direction of the distal end 7.

Thus, as illustrated in Fig. 2, all of the second bore 20 formed in the second region 21 and the bores formed in the distal region D can be positioned to correspond to the lower tibia 2000. In addition, with this bore arrangement, the second bore 20 formed in the second region 21 can couple the upper portion of the lower tibia 2000 with the intramedullary nail 2, and the bores (e.g., 36, 37, 38 and 39 in Fig. 2) formed in the distal region D can couple the lower portion of the lower tibia 2000 with the intramedullary nail 2. Preferably, the above coupling may be a bolt coupling.

As such, when both the upper and lower portions of the lower tibia 2000 are coupled with the intramedullary nail 2, the lower tibia 2000 is supported by at least two points in the longitudinal direction. With this supporting method, tilting of the lower tibia 2000 caused by the muscles around the lower tibia 2000 can be prevented since the lower tibia 2000 cannot be rotated.

Furthermore, since the number of second bore 20 formed in the second region 21 and the number of the bore formed in the distal region D may be plural, the upper and lower portions of the lower tibia 2000 can be fixed to the intramedullary nail 2 more firmly, thereby even a minute tilting of the lower tibia 2000 can be prevented.

As such, if the tiling of the upper tibia 1000 and the lower tibia 2000 is prevented, the bone can be guided in an intended direction until the bone formation is completed and the segmented bone is finally bonded.

In addition, it is possible to minimize complications caused by the tilting of the upper tibia 1000 and the lower tibia 2000, avoid reoperation, and allow the bonded bone to properly support the body weight, thereby degradation of the motor ability can be minimized.

Preferably, the range of the first region 11 is 0.17L to 0.4L from the proximal end, and the range of the second region 21 is 0.51L to 0.8L from the proximal end.

In this case, the first bore 10 is further away from the bore formed in the proximal region P and the second bore 20 is further away from the bore formed in the distal region D. Consequently, the distance between the two supporting points of the 2-point support becomes longer. Thus, the resistance against the rotational force applied by the peripheral muscles can be further increased, even if the same number of bores and bolts are used to couple the tibia to the intramedullary nail 2.

Further, as the resistance against the rotational force is increased, the number of the first bore 10, the second bore 20 and the coupling members (e.g., bolts) for coupling and supporting the upper tibia 1000 and the lower tibia 2000 with the intramedullary nail 2 to prevent tilting can be minimized.

In addition, when the number of the first bore 10, the second bore 20, and the bolts is minimized, operation time and operation cost can be reduced, and the coupling member such as the bolt that invades the medullary cavity can be minimized, thereby the recovery rate of an affected area can also be promoted.

The intramedullary nail 2 may have a through hole 6 formed to extend from the proximal end 4 to the distal end 7 along the central axis 5. By forming the through hole 6, bone formation for complete bonding of the bones can be promoted. Also, the first bore 10 and the second bore 20 may be formed to communicate with the through hole. Then, the intramedullary nail 2 can be coupled with the bone more easily using a coupling member (e.g., a bolt).

All the bores that may be formed in the intramedullary nail 2 including the first bore 10 and the second bore 20 may be formed radially with respect to the central axis. When the bores are formed radially, it is possible to prevent the intramedullary nail 2 from being displaced upward and downward in the medullary cavity, effectively resist the forces in various directions applied by the muscles around the bone, and couple the intramedullary nail 2 with the bone more easily.

At least one bore among the bores including the first bore 10 and the second bore 20 may be formed to have a predetermined angle with respect to the proximal end face. That is, for example, a bore 33 may have a predetermined angle, as illustrated in Fig. 3, such that an extension of the bore 33 meets a reference line R, which is an extension of the proximal end 4. Patients take various actions such as walking, with the intramedullary nail 2 inserted into the bone. If one or more bores are formed to have a certain angle with respect to the proximal end face, the impact and load applied in various directions does not displace the intramedullary nail 2 in the medullary cavity and the initial coupling state with the bone can be maintained. Thus, even a minute tilting of the bone can be prevented, and the bone can be guided in an intended direction until the segmented bone is finally bonded.

Two or more of the bores including the first bore 10 and the second bore 20 may be formed so that they are not parallel to each other. For example, the bore 33 and the first bore 10 shown in Fig. 3 are formed so as not to be parallel to each other. This feature makes it possible to more easily and effectively prevent fine tilting due to the impact or load applied in various directions.

At least one bore among the bores including the first bore 10 and the second bore 20 may be a Y-shaped bore forming three openings on the outer circumferential surface of the intramedullary nail. For example, in Figs. 2 and 3, the bore 39 has three openings towards the outer surface of the nail. Since such a Y-shaped bore can accommodate coupling members (e.g., bolts) in three directions, or can accommodate two or more coupling members when a coupling member having a bore formed therein is used, the intramedullary nail 2 and the bone can be coupled more securely. Therefore, the coupling between the intramedullary nail 2 and the bone becomes more secure, and it is also possible to more easily and effectively prevent fine tilting due to impact or load applied in various directions.

At least one of the bores including the first bore 10 and the second bore 20 may be a bore elongated in the longitudinal direction. For example, in Figs. 2 and 3, the bore 34 has a longitudinally elongated shape. If a bore is elongated in the longitudinal direction, it will have a margin to receive a coupling member (e.g., a bolt) and will give more flexibility in positioning when an intramedullary nail 2 is inserted into the bone and coupled thereto.

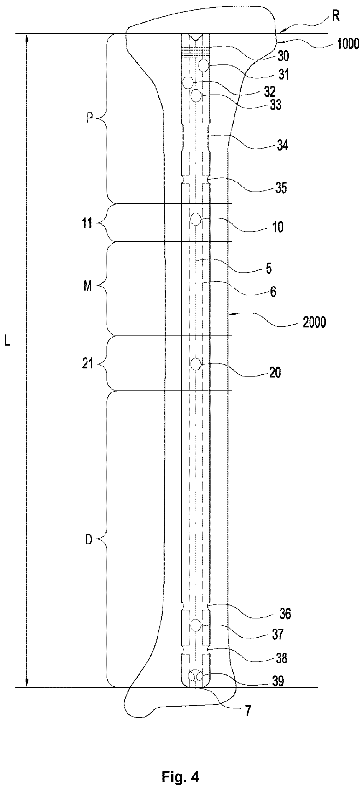

Fig. 4 illustrates a boned bone after completion of bone formation without tilting of the segmented bone due to the action of peripheral muscles, with the intramedullary nail of the present invention inserted into the bone.

That is, with the intramedullary nail 2 of the present invention, the upper tibia 1000 and the lower tibia 2000 are guided and bonded in an intended direction, and complications during bonding of the segmented bone or distraction osteogenesis are minimized. Also, the bonded bone can support the body weight properly, and at the same time, the degradation of motor ability can be minimized.

It will be apparent to those skilled in the art that the foregoing explanation is to show exemplary embodiments of the invention and that various modifications and variations can be made in the present invention without departing from the spirit or scope of the invention. It is, therefore, to be understood that the above-described embodiments are illustrative in all aspects and not restrictive. For example, each component described as a single entity may be distributed and implemented, and components described as being distributed may also be implemented in a combined form.

The scope of the present invention is defined by the appended claims, and all changes or modifications derived from the meaning and scope of the claims and their equivalents should be interpreted as being included in the scope of the present invention.

### EXPLANATION OF REFERENCE NUMBERS

| | |
|---|---|
| P: proximal region | 33: bore |
| M: central region | 34: bore |
| D: distal region | 35: bore |
| 1: conventional intramedullary nail | 36: bore |
| 2: intramedullary nail | 37: bore |
| 4: proximal end | 38: bore |
| 5: central axis | 39: bore |
| 6: through hole | 100: upper tibia |
| 7: distal end | 200: lower tibia |
| 10: first bore | 101: bolt |
| 11: first region | 201: bolt |
| 20: second bore | 300: hard part |
| 21: second region | 301: medullary cavity |
| 30: bore | 302: muscles |
| 31: bore | 1000: upper tibia |
| 32: bore | 2000: lower tibia |

## Claims

1. An intramedullary nail having an overall length of L, the nail comprising
one or more bores formed in each of a proximal region located in an upper portion of the intramedullary nail and a distal region located in a lower portion of the intramedullary nail,
wherein one or more first bores are formed in a first region located on a lower side of the proximal region and a range of the first region is 0.12L to 0.45L from a proximal end of the intramedullary nail, and
wherein one or more second bores are formed in a second region located on an upper side of the distal region and a range of the second region is between 0.46L to 0.85L from the proximal end.

2. The intramedullary nail of claim 1, wherein the range of the first region is 0.17L to 0.4L from the proximal end, and the range of the second region is 0.51L to 0.8L from the proximal end.

3. The intramedullary nail of claim 1 or 2, wherein the intramedullary nail has a through hole formed to extend from the proximal end toward a distal end of the intramedullary nail, and the first bore and the second bore are configured to communicate with the through hole.

4. The intramedullary nail of claim 1 or 2, wherein the bores including the first bore and the second bore are formed radially with respect to a central axis.

5. The intramedullary nail of claim 1 or 2, wherein at least one of the bores including the first bore and the second bore is formed to have a predetermined angle with respect to a face of the proximal end.

6. The intramedullary nail of claim 1 or 2, wherein at least two of the bores including the first bore and the second bore are formed so as not to be parallel to each other.

7. The intramedullary nail of claim 1 or 2, wherein at least one of the bores including the first bore and the second bore is a Y-shaped bore forming three openings on an outer circumferential surface of the intramedullary nail.

8. The intramedullary nail of claim 1 or 2, wherein at least one of the bores including the first bore and the second bore is a bore having a longitudinally-elongated shape.
